# EUROPÄISCHE PATENTSCHRIFT

(11) **EP 3 265 154 B1**
(45) Veröffentlichungstag und Bekanntmachung des Hinweises auf die Patenterteilung: **18.11.2020**
(21) Anmeldenummer: 16707143.0
(22) Anmeldetag: 02.03.2016
(51) Int. Cl.: A61M 5/315, A61M 5/28

(54) **KOLBENSTANGE MIT WENIGSTENS DREI RINGELEMENTEN FÜR EINE VORGEFÜLLTE SPRITZE**
PLUNGER ROD COMPRISING AT LEAST THREE ANNULAR ELEMENTS FOR A PREFILLED SYRINGE
TIGE DE PISTON COMPRENANT AU MOINS TROIS ÉLÉMENTS CYLINDRIQUES POUR UNE SERINGUE PRÉREMPLIE

(30) Priorität: 02.03.2015 EP 15000587
(43) Veröffentlichungstag der Anmeldung: 10.01.2018
(60) Teilanmeldung: 20186074.9
(73) Patentinhaber: Fresenius Kabi Austria GmbH, 8055 Graz (AT)
(72) Erfinder: BALDAUF, Wolfgang, 8072 Fernitz (AT); HEINRICH, Jörg, 82031 Grünwald (DE); ZAUNER, Christoph, 8401 Kalsdorf bei Graz (AT)
(74) Vertreter: Fresenius Kabi Deutschland GmbH
(86) Internationale Anmeldenummer: PCT/EP2016/054351
(87) Internationale Veröffentlichungsnummer: WO 2016/139215

(56) Entgegenhaltungen:
- EP-A1- 2 484 393
- WO-A1-01/97885
- WO-A1-2010/139793
- WO-A1-2014/122782
- JP-A- 2002 272 843
- JP-A- 2011 072 394

## Beschreibung

Die Erfindung betrifft eine Kolbenstange für einen mit einer medizinischen Flüssigkeit vorgefüllten Spritzenkörper sowie eine Spritze umfassend die Kolbenstange und den mit der medizinischen Flüssigkeit vorgefüllten Spritzenkörper.

Aus der WO 2014/053560 A1 ist eine mit einer medizinischen Flüssigkeit vorgefüllte Spritze bekannt. Die Kolbenstange und der Spitzenkörper sind in einer Ausführungsform nebeneinander liegend in einer Überverpackung gelagert. Zur Anwendung der Spritze werden zunächst die Kolbenstange und der Spritzenkörper aus der Überverpackung entnommen. Dann wird die Kolbenstange in den die Rückseite des Spritzenkörpers verschließenden Kolben eingeschraubt. Der Inhalt der genannten Patentanmeldung wird vollumfänglich in die vorliegende Patentanmeldung durch Bezugnahme inkorporiert.

Die WO 2010/139793 A1 offenbart eine vorgefüllte zwei-Kammernspritze, aufweisend einen hinteren Kolben in den einen Kolbenstange einführbar ist. Zur Verbindung der Kolbenstange mit dem hinteren Kolben weißt die Kolbenstange an dem Ende, welches in Eingriff mit dem hinteren Kolben gebracht wird, umlaufende Verzahnungen auf.

Aus der WO 2014/122782 A1 ist eine mit einer medizinischen Flüssigkeit vorgefüllte Spritze bekannt. Die Kolbenstange weist in einer Ausführungsform an ihrem proximalen Ende Ringelemente auf, die durch Flügelelemente verbunden sind.

WO01/97885, JP2011072394, and JP2002272843 offenbart eine Spritze mit einer Kolbenstange mit zwei Ringelementen.

Der Erfindung liegt die Aufgabe zugrunde, eine verbesserte Kolbenstange bereitzustellen. Das Einsetzen, insbesondere das Einschrauben, der Kolbenstange in den Kolben soll verbessert werden. Insbesondere soll eine Leckage durch ein Verkippen des Kolbens möglichst vermieden werden. Ferner soll die Führung der Kolbenstange in dem Spritzenkörper verbessert werden, insbesondere bei der Verwendung der Spritze in einer Spritzenpumpe. Diese Aufgabe wird durch Gegenstände mit den Merkmalen nach den unabhängigen Ansprüchen gelöst. Vorteilhafte Ausführungsformen sind Gegenstand der abhängigen Ansprüche, der Beschreibung sowie der Zeichnungen.

Die erfindungsgemäße vorgefüllte Spritze umfasst die folgenden Bestandteile: einen mit einer medizinischen Flüssigkeit befüllten Spritzenkörper, der an einer Vorderseite eine Düse aufweist, die mit einer Kappe verschlossen ist, und der an einer Rückseite mit einem verschiebbaren Kolben verschlossen ist, und eine über die Rückseite in den Spritzenkörper einführbare Kolbenstange, die an einer Vorderseite einen Verbindungsabschnitt aufweist, über den die Kolbenstange mit dem Kolben verbindbar ist, wobei an einer Rückseite des Verbindungsabschnitts wenigstens drei Ringelemente, die sich zumindest abschnittweise um eine Längsachse der Kolbenstange erstrecken, so an der Kolbenstange angeordnet sind, dass sie sich, wenn die Kolbenstange vollständig mit dem Kolben verbunden ist, in dem Spritzenkörper befinden, wobei ein Außendurchmesser (D) der drei Ringelemente gleich einem Innendurchmesser des Spritzenkörpers ist oder etwas kleiner als ein Innendurchmesser des Spritzenkörpers ist, wobei zwischen den Ringelementen über den Umfang der Kolbenstange verteilte Flügelelemente angeordnet sind, welche sich radial nach außen erstrecken, die Ringelemente miteinander verbinden und einen Außendurchmesser (D_{f}) aufweisen, der kleiner als ein Außendurchmesser (D) der Ringelemente ist. Die Spritze ist dabei in ihrem initialen Zustand. D.h. der Kolben wurde noch nicht bewegt zum Ausstoßen der Flüssigkeit.

Die drei Ringelemente unterstützen ein koaxiales Verbinden der Kolbenstange mit dem Kolben. Die Ringelemente erweisen sich zum einen als vorteilhaft, da sie ein Verkippen über den gesamten Umfang von 360° verhindern. Zum anderen ermöglichen die drei Ringelemente dem Anwender beim Verbinden der Kolbenstange mit dem Kolben eine haptische und/oder akustische Kontrolle. Verbindet beispielsweise der Anwender die Kolbenstange ordnungsgemäß gerade mit dem Kolben, bekommt er keine haptische und/oder akustische Rückmeldung. Versucht der Anwender dagegen unbeabsichtigt die Kolbenstange nicht ordnungsgemäß schief mit dem Kolben zu verbinden, so kann er insbesondere den Übergang der Kolbenstange von dem mittleren Ringelement zu dem hinteren Ringelement an der Kante der hinteren Spritzenkörperöffnung als eine Art Sprung, vorzugsweise verbunden mit einer Art Klicken wahrnehmen. Der Anwender kann dadurch erkennen, dass er versucht, die Kolbenstange nicht ordnungsgemäß mit dem Kolben zu verbinden und daraufhin die Lage der Kolbenstange entsprechend korrigieren. Vorzugsweise sind die Position des Kolbens in dem vorgefüllten Spritzenkörper und die Position der drei Ringelemente an der Kolbenstange so aufeinander abgestimmt, dass in einem ersten Schritt, wenn die Kolbenstange mit ihrem Verbindungsabschnitt an dem Kolben angesetzt wird, bereits die beiden vorderen Ringe im Inneren des Spritzenkörpers angeordnet sind. Beim Verbinden, beispielsweise Einschrauben, wird die Kolbenstange zunächst durch die beiden vorderen Ringelemente geführt. Der hintere dritte Ring unterstützt die Führung der Kolbenstange beim finalen, festen Verbinden der Kolbenstange mit dem Kolben.

Spätestens sind die drei Ringelemente in dem Zustand, in dem die Kolbenstange vollständig mit dem Kolben verbunden ist, vorzugsweise in den Kolben eingeschraubt, ist, im Inneren des Spritzenkörpers positioniert.

Erfindungsgemäß ist ein Außendurchmesser der drei Ringelemente gleich einem Innendurchmesser des Spritzenkörpers oder etwas kleiner als ein Innendurchmesser des Spritzenkörpers. Dadurch können die drei Ringelemente beim Verbinden der Kolbenstange mit dem Kolben und später beim Bewegen des Kolbens zum Ausstoßen der Flüssigkeit auf einer Innenseite des Spritzenkörpers geführt werden. Vorzugsweise ist der Außendurchmesser der drei Ringelemente gleich.

Jeweils zwischen den Ringelementen sind über den Umfang der Kolbenstangen verteilte Flügelelemente oder Rippen angeordnet, welche sich radial nach außen erstrecken. Vorzugsweise verbinden die Flügelelemente die Ringelemente miteinander. Die Flügelelemente erweisen sich als vorteilhaft, da sie eine längere, abschnittsweise kontinuierliche Führung entlang der Längsachse der Kolbenstange ermöglichen.

In einer Ausgestaltung sind, insbesondere jeweils, zwischen den Ringelementen mindestens vier Flügelelemente, vorzugsweise unter einem Winkel von 90° zueinander, angeordnet. Dadurch soll eine möglichst koaxiale Führung der Kolbenstange unterstützt werden. Vorzugsweise weisen die Flügelelemente einen Außendurchmesser auf, der gleich dem Innendurchmesser des Spritzenkörpers ist oder etwas kleiner ist als der Innendurchmesser des Spritzenkörpers. Die Flügelelemente besitzen einen Außendurchmesser, der kleiner als ein Außendurchmesser der Ringelemente ist. Dadurch wird beim Verbinden der Kolbenstange mit dem Kolben und/oder beim Einführen der Kolbenstange in den Spritzenkörper die haptische und/oder akustische Kontrolle verbessert. In einer Ausgestaltung ist der Außendurchmesser der Flügelelemente gegenüber dem Außendurchmesser der Ringelemente um 1 mm bis 10 mm, vorzugsweise um 4 mm bis 8 mm reduziert.

Die vorgefüllte Spritze kann zum Beispiel in einer Überverpackung bereitgestellt werden. Ist zum Beispiel die medizinische Flüssigkeit sauerstoffempfindlich und der Spritzenkörper nicht ausreichend sauerstoffimpermeabel, so kann die Spritze in einer sauerstoffimpermeablen Überverpackung verpackt sein, zum Beispiel in einem Blister. Die Kolbenstange kann zum Beispiel bereits an dem Kolben vormontiert sein. Sie kann aber auch nicht vormontiert neben dem Spritzenkörper in der Überverpackung liegen. Daher liegt im Bereich der Erfindung auch eine Überverpackung mit einem Innenraum, in dem die vorstehend beschriebene erfindungsgemäße Spritze eingeschlossen ist.

Weiterhin liegt im Bereich der Erfindung auch die Kolbenstange, insbesondere für eine oder für die vorstehend genannte vorgefüllte Spritze. Die Kolbenstange umfasst einen vorderseitigen Verbindungsabschnitt, über den die Kolbenstange mit einem Kolben verbindbar ist. Dabei sind an einer Rückseite des Verbindungsabschnitts wenigstens drei Ringelemente angeordnet, welche sich zumindest abschnittweise um eine Längsachse der Kolbenstange erstrecken. Die drei Ringelemente sind, vorzugsweise jeweils, in einem Abstand R zueinander angeordnet sind mit 0,5 mm ≤ R ≤ 20mm, bevorzugt 1 mm ≤ R ≤ 10mm, besonders bevorzugt 2 mm ≤ R ≤ 8mm. Die Ringelemente besitzen beispielsweise eine Dicke von etwa 0,5 mm bis etwa 5 mm, vorzugsweise von etwa 1 mm bis etwa 3 mm. Die Kolbenstange besitzt einen Durchmesser D mit 8 mm ≤ D ≤ 30 mm und/oder eine Länge L mit 80 mm ≤ L ≤ 150 mm.

Ferner wird auch noch eine Spritze beansprucht, umfassend einen Spritzenkörper, einen Kolben und die vorstehend beschriebene Kolbenstange, wobei der Kolben so in dem Spritzenkörper positionierbar ist und die drei Ringelemente so an der Kolbenstange angeordnet sind, dass sie sich, wenn die Kolbenstange vollständig mit dem Kolben verbunden ist, in dem Spritzenkörper befinden. Vorzugsweise ist der Spritzenkörper mit einer medizinischen Flüssigkeit vorgefüllt.

Die vorgefüllte Spritze kann zum Beispiel ein Fassungsvolumen von 5 ml bis 100 ml haben. Die medizinische Flüssigkeit kann beispielsweise eine Flüssigkeit zur enteralen und/oder parenteralen Ernährung und/oder zur Infusion sein oder umfassen. Die medizinische Flüssigkeit kann durch eine Lösung und/oder durch eine Emulsion bereitgestellt werden. Die medizinische Flüssigkeit kann auch medizinische Wirkstoffe enthalten. Gemäß einer Ausführungsform ist oder umfasst die medizinische Flüssigkeit das Arzneistofffluid Propofol, insbesondere eine Propofol-Emulsion. Propofol wird beschrieben durch den chemischen Namen 2,6-Diisopropylphenol (IUAPC).

Der Spritzenkörper kann aus Kunststoff geformt sein, welcher eines der folgenden Polymere umfasst: Cyclo-Olefin-Copolymer, Cyclo-Olefin-Polymer oder Crystal Clear Polymer. Ein derartiger Kunststoffbehälter ist gegen Lösungsmittel widerstandsfähig. Insbesondere kann ein derartiger Kunststoffbehälter zur Lagerung von Propofol, das als Lösungsmittel wirkt, verwendet werden. Vorzugsweise sind die Außenseite des Kolbens und/oder die Innenseite des Spritzenkörpers zumindest abschnittsweise mit einem Gleitmittel bedeckt, vorzugsweise silikonisiert.

Gemäß einer Ausführungsform ist die Kunststoffkolbenstange aus Kunststoff geformt, welcher vorzugsweise eines der folgenden Polymere umfasst: Cyclo-Olefin-Copolymer, Cyclo-Olefin-Polymer oder Crystal Clear Polymer, oder sie ist aus Polypropylen geformt.

Die Erfindung wird nachfolgend anhand von Ausführungsbeispielen in Verbindung mit den Zeichnungen im Einzelnen beschrieben werden.

Es zeigen:

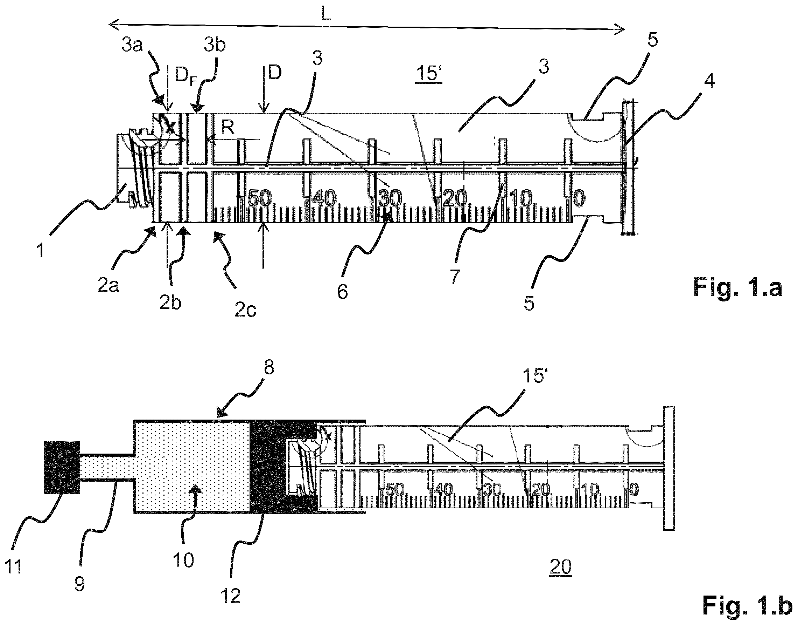

- Fig. 1.a: eine Seitenansicht einer Kolbenstange;
- Fig. 1.b: eine Seitenansicht einer erfindungsgemäßen vorgefüllten Spritze mit der Kolbenstange aus Figur 1.a;
- Fig. 2.a: eine Seitenansicht einer Kolbenstange gemäß einer Ausführungsform der Erfindung;

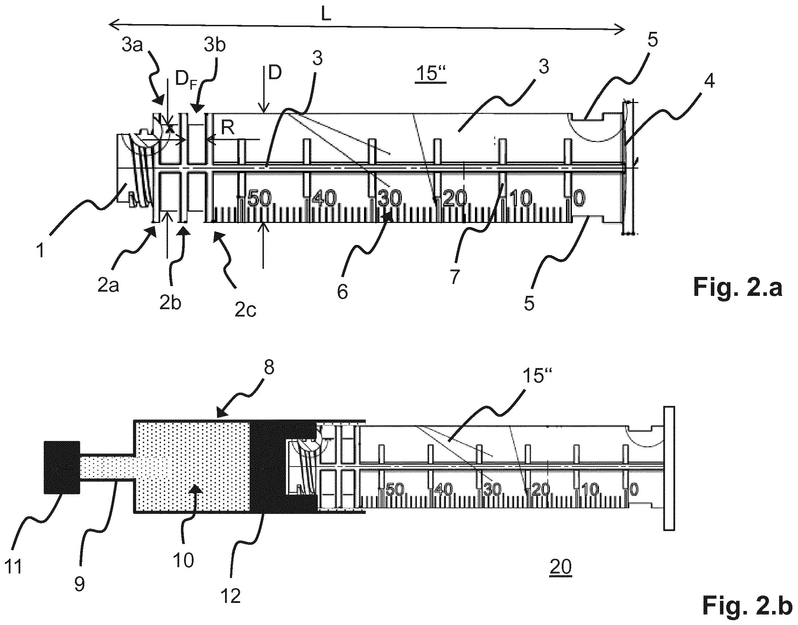

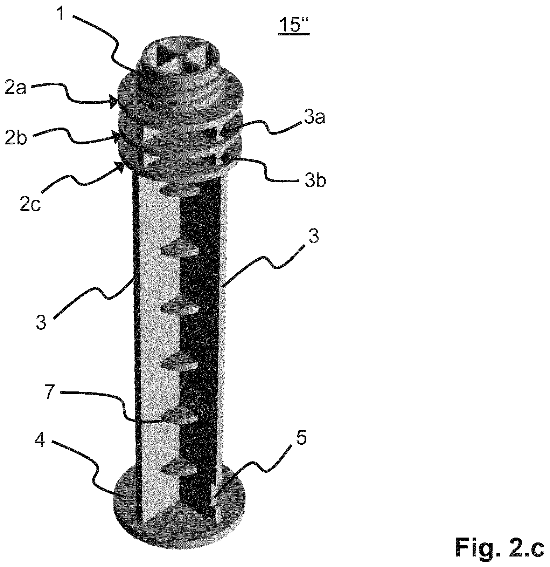

- Fig. 2.b: eine Seitenansicht einer erfindungsgemäßen vorgefüllten Spritze mit der Kolbenstange aus Figur 2.a;
- Fig. 2.c: eine perspektivische Ansicht der Kolbenstange gemäß der Ausführungsform der Erfindung aus Figur 2.a;

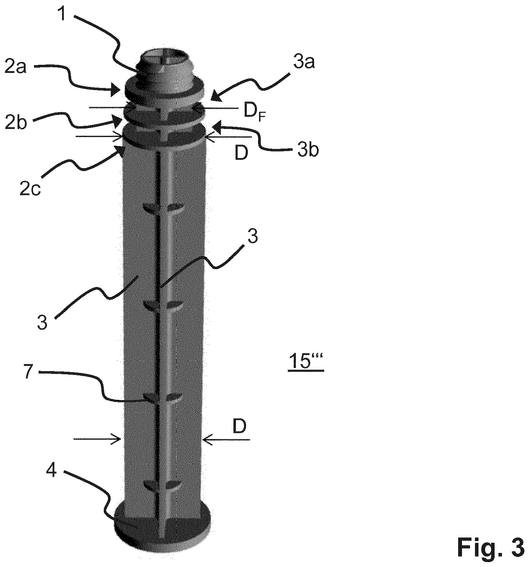

- Fig. 3: eine perspektivische Ansicht einer Kolbenstange gemäß einer zweiten Ausführungsform der Erfindung und

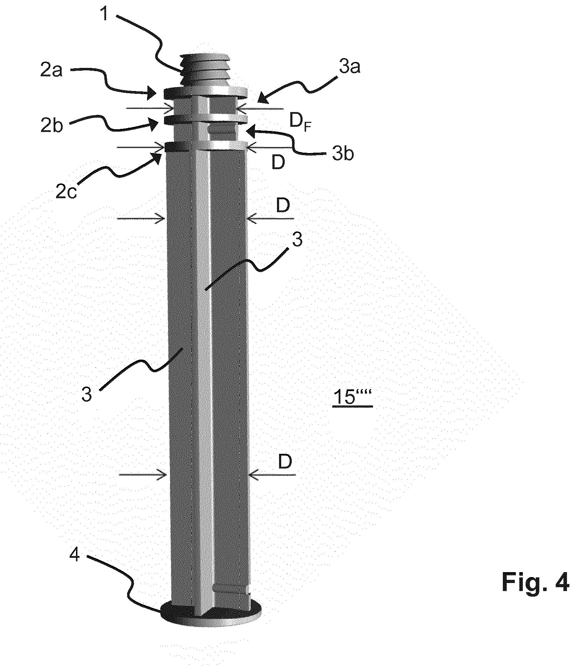

- Fig. 4: eine perspektivische Ansicht einer Kolbenstange gemäß einer dritten Ausführungsform der Erfindung;

Figur 1.a zeigt eine Kolbenstange 15' gemäß einer Ausführungsform. Die Kolbenstange 15' besitzt einen im Wesentlichen kreuzförmigen Querschnitt und wird durch die beiden Schenkel 3 gebildet. Entlang der Längsachse sind Stabilisierungselemente 7 angebracht. Die Rückseite der Kolbenstange 15' wird durch einen im Durchmesser größeren Flansch 4 abgeschlossen. Die am hinteren Ende eingebrachten Einkerbungen 5 ermöglichen einen Betrieb der Kolbenstange 15' (als Bestandteil einer Spritze 20) in einer Spritzenpumpe. Die Kolbenstange 15' ist ausgelegt für den Betrieb in einer Spritze 20 mit einem Fassungsvolumen von bis zu etwa 50 ml bis 70 ml. Auf der Kolbenstange 15' ist eine Skala 6 bis 50 ml angegeben. Die Kolbenstange 15' besitzt einen Durchmesser D mit 25 mm ≤ D ≤ 30 mm und/oder eine Länge L mit 100 mm ≤ L ≤ 150 mm.

Die Vorderseite der Kolbenstange 15' wird bereitgestellt durch einen Verbindungsabschnitt 1, über den die Kolbenstange 15' mit einem hier nicht dargestellten Kolben 12 verbunden wird (siehe dazu Figur 1.b). Der Verbindungsabschnitt 1 wird hier beispielhaft durch ein Schraubgewinde bereitgestellt. An der Rückseite des Verbindungsabschnitts 1 schließen sich drei Ringelemente 2a, 2b, 2c an. Dadurch kann insbesondere dem Anwender beim Einschrauben der Kolbenstange 15" in den Kolben 12 eine haptische und/oder akustische Kontrolle ermöglicht werden. Vorzugsweise erstrecken sich die Ringelemente 2a, 2b, 2c vollständig über den Umfang der Kolbenstange 15'. Das vordere Ringelement 2a bildet zudem den Anschlag beim Einschrauben der Kolbenstange 15' in den Kolben 12. Vorzugsweise sind die drei Ringelemente 2a, 2b, 2c äquidistant zueinander angeordnet. In einer Ausführungsform sind die drei Ringelemente 2a, 2b, 2c jeweils in einem Abstand R von 2 mm bis 8 mm zueinander angeordnet. Die Ringelemente 2a, 2b, 2c besitzen beispielsweise eine Dicke von etwa 0,5 mm bis etwa 5 mm, vorzugsweise von etwa 1 mm bis etwa 3 mm.

Der kreuzförmige Querschnitt der Kolbenstange 15' setzt sich auch zwischen den drei Ringelementen 2a, 2b, 2c fort. Unter Vernachlässigung des Flansches 4 und des Verbindungsabschnitts 1 ist der Durchmesser D der Kolbenstange entlang der Längsachse gleich oder im Wesentlichen gleich. Dadurch werden zwischen den drei Ringelementen 2a, 2b, 2c sogenannte Flügelelemente 3a, 3b oder Rippen gebildet. Die vorderen, hier vier, Flügelelemente 3a verbinden den vorderen Ring 2a mit dem mittleren Ring 2b. Die hinteren, hier vier, Flügelelemente 3b verbinden den mittleren Ring 2b mit dem hinteren Ring 2b. Die Ringelemente 2a, 2b, 2c und die Flügelelemente 3a, 3b ermöglichen ein möglichst koaxiales Einführen der Kolbstange 15' in den Spritzenkörper 8 und dadurch ein möglichst koaxiales Einschrauben der Kolbstange 15' in den Kolben 12 (siehe dazu Figur 1.b). Ein schräges Ansetzen der Kolbenstange 15' an dem Kolben 12, ein damit verbundenes mögliches Verkippen des Kolbens 12 und eine letztendlich daraus resultierende Leckage können dadurch reduziert oder sogar vermieden werden.

Figur 1.b illustriert die Verwendung der Kolbenstange 15' aus Figur 1.a in einer vorgefüllten Spritze 20. Die Spritze 20 umfasst einen Spritzenkörper 8 mit einer an der Vorderseite des Spritzenkörpers 8 angeordneten Düse 9, eine die Düse 9 verschließende Kappe 11, einen im Spritzenkörper 8 angeordneten Kolben 12, welcher den Innenraum des Spritzenkörpers 8 flüssigkeitsdicht verschließt, und die Kolbenstange 15' aus Figur 1a, welche über ihren Verbindungsabschnitt 1 mit dem Kolben 12 verbunden ist. Die Verbindung zwischen dem Kolben 12 und der Kolbenstange 15' wird hier durch eine Schraubverbindung bereitgestellt. Entsprechend ist auch im Inneren des Kolbens 12 ein Gewinde vorgesehen, welches aber in der Figur nicht dargestellt ist.

Der Außendurchmesser D bzw. D_{F} der Kolbenstange 15' und/oder der Ringelemente 2a, 2b, 2c und/oder der Flügelelemente 3a, 3b kann gleich dem Innendurchmesser des Spritzenkörpers 8 sein. Um eine vereinfachte Bewegung des Kolbenstange 15' in dem Spritzenkörper 8 zu ermöglichen, ist insbesondere der Außendurchmesser D bzw. D_{F} der Kolbenstange 15' und/oder der Ringelemente 2a, 2b, 2c und/oder der Flügelelemente 3a, 3b kleiner als der Innendurchmesser des Spritzenkörpers 8. Vorzugsweise ist der Außendurchmesser D bzw. D_{F} um etwa 0,5 mm bis 5 mm reduziert gegenüber dem Innendurchmesser des Spritzenkörpers 8.

Die vorgefüllte Spritze 20 kann zum Beispiel über Ihre zunächst offene Rückseite befüllt und dann mit dem Kolben 12 verschlossen werden. Die Kolbenstange 15' kann dann zu einem späteren Zeitpunkt mit dem Kolben 12 verbunden werden, zum Beispiel kurz vor der Anwendung. Der Kolben 12 kann aber auch zum Beispiel mit bereits in den Kolben 12 eingeschraubter Kolbenstange 15' in den Spritzenkörper 8 eingebracht werden.

Die Position des Kolbens 12 in dem vorgefüllten Spritzenkörper 8 und die Position der drei Ringelemente 2a, 2b, 2c an der Kolbenstange 15' sind vorzugsweise so aufeinander abgestimmt, dass in einem ersten Schritt, wenn die Kolbenstange 15' mit ihrem Verbindungsabschnitt 1 an dem Kolben 12 angesetzt wird, bereits die beiden vorderen Ringe 2a, 2b im Inneren des Spritzenkörpers 8 angeordnet und vorzugsweise durch die Innenseite des Spritzenkörpers 8 geführt sind. Beim Einschrauben wird die Kolbenstange 15' zunächst durch die beiden vorderen Ringelemente 2a, 2b und die vorderen Flügelelemente 3a und dann auch durch die hinteren Flügelelemente 3b in dem Spritzenkörper 8 geführt. Der hintere dritte Ring 2c unterstützt die Führung der Kolbenstange 15' beim finalen, festen Verbinden der Kolbenstange 15' mit dem Kolben 12. Spätestens sind die drei Ringe 2a, 2b, 2c in dem Zustand, in dem die Kolbenstange 15' vollständig mit dem Kolben 12 verbunden ist, vorzugsweise in den Kolben 12 eingeschraubt, ist, im Inneren des Spritzenkörpers 8 positioniert. Die Ringelemente 2a, 2b, 2c erweisen sich als vorteilhaft, da sie ein Verkippen über den gesamte Umfang von 360° verhindern. Die Flügelelemente 3a, 3b erweisen sich als vorteilhaft, da sie eine längere Führung entlang der Längsachse der Kolbenstange 15' ermöglichen. Die vorliegende Erfindung verbindet diese Vorteile miteinander.

Figur 2.a bis 2.c zeigen eine Kolbenstange 15" gemäß einer Ausführungsform der Erfindung. Nachfolgend werden nur die Unterschiede zu der Kolbenstange 15' aus Figur 1.a erläutert. Für alle anderen Komponenten wird auf die vorstehende Beschreibung zu Figur 1.a verwiesen. Auf der Kolbenstange 15" ist keine Skala angegeben. Im Unterschied zu der in Figur 1.a dargestellten Ausführungsform besitzen die Flügelelemente 3a, 3b, welche die drei Ringelemente 2a, 2b, 2c miteinander verbinden, nicht den gleichen Außendurchmesser wie die drei Ringelemente 2a, 2b, 2c. Der Außendurchmesser D_{F} der Flügelelemente 3a, 3b ist kleiner als der Außendurchmesser D der drei Ringelemente 2a, 2b, 2c. Vorzugsweise ist der Außendurchmesser D_{F} der Flügelelemente 3a, 3b um etwa 4 mm bis 8 mm reduziert gegenüber dem Außendurchmesser D der Ringelemente 2a, 2b, 2c. In dem vorliegenden Fall sind es genau drei Ringelemente, die im vorderen Bereich der Kolbenstange 15' liegen.

Dadurch kann insbesondere dem Anwender beim Einschrauben der Kolbenstange 15" in den Kolben 12 eine haptische und/oder akustische Kontrolle ermöglicht werden. Schraubt der Anwender die Kolbenstange 15" ordnungsgemäß gerade in den Kolben 12 ein, bekommt er keine haptische und/oder akustische Rückmeldung. Versucht der Anwender dagegen die Kolbenstange 15" unbeabsichtigt und nicht ordnungsgemäß schief in den Kolben 12 einzuschrauben, so kann er insbesondere den Übergang der Kolbenstange 15" von dem mittleren Ringelement 2b zu dem hinteren Ringelement 2c an der Kante 14 in der hinteren Spritzenkörperöffnung (siehe dazu Figur 2.b) als eine Art Sprung, vorzugsweise verbunden mit einer Art Klicken wahrnehmen. Der Anwender kann dadurch erkennen, dass er versucht, die Kolbenstange 15" nicht ordnungsgemäß mit dem Kolben 12 zu verbinden und daraufhin die Lage der Kolbenstange 15" entsprechend korrigieren.

Weiterhin zeigt Figur 3 eine Kolbenstange 15'" gemäß einer zweiten Ausführungsform der Erfindung. Der Aufbau dieser Kolbenstange 15'" entspricht im Wesentlichen dem Aufbau der Kolbenstange 15" aus den Figur 2.a bis 2.c. Die vorliegende Kolbenstange 15'" weist lediglich andere Abmessungen auf, da sie für eine Spritze 20 mit einem Fassungsvolumen von bis zu etwa 20 ml bis 30 ml ausgelegt ist. Die Kolbenstange 15'" besitzt einen Durchmesser D mit 13 mm ≤ D ≤ 23 mm und/oder eine Länge L mit 100 mm ≤ L ≤ 150 mm. Der Abstand R der Ringelemente beträgt auch hier 2 mm ≤ R ≤ 8 mm.

Abschließend zeigt Figur 4 eine Kolbenstange 15"" gemäß einer dritten Ausführungsform der Erfindung. Der Aufbau dieser Kolbenstange 15"" entspricht im Wesentlichen dem Aufbau der Kolbenstangen 15" und 15'" aus den Figuren 2.a bis 2.c und 3. Die vorliegende Kolbenstange 15"" weist lediglich andere Abmessungen auf, da sie für eine Spritze 20 mit einem Fassungsvolumen von bis zu etwa 10 ml bis 15 ml ausgelegt ist. Die Kolbenstange 15"" besitzt einen Durchmesser D mit 8 mm ≤ D ≤ 18 mm und/oder eine Länge L mit 80 mm ≤ L≤ 110 mm. Der Abstand R der Ringelemente beträgt hier 2 mm ≤ R ≤ 8 mm. Zudem sind an der Kolbenstange 15"" keine Stabilisierungselemente 7 vorgesehen.

Es ist dem Fachmann ersichtlich, dass die beschriebenen Ausführungsformen beispielhaft zu verstehen sind. Die Erfindung ist nicht auf diese beschränkt sondern kann in vielfältiger Weise variiert werden, ohne das Wesen der Erfindung zu verlassen. Merkmale einzelner Ausführungsformen und die im allgemeinen Teil der Beschreibung genannten Merkmale können jeweils untereinander als auch miteinander kombiniert werden.

## Patentansprüche

1. Vorgefüllte Spritze (20) umfassend
- einen mit einer medizinischen Flüssigkeit (10) befüllten Spritzenkörper (8), der an einer Vorderseite eine Düse (9) aufweist, die mit einer Kappe (11) verschlossen ist, und der an einer Rückseite mit einem verschiebbaren Kolben (12) verschlossen ist, und
- eine über die Rückseite in den Spritzenkörper (8) einführbare Kolbenstange (15', 15", 15"', 15""), die an einer Vorderseite einen Verbindungsabschnitt (1) aufweist, über den die Kolbenstange (15', 15", 15'", 15"") mit dem Kolben (12) verbindbar ist, wobei
an einer Rückseite des Verbindungsabschnitts (1) wenigstens drei Ringelemente (2a, 2b, 2c), die sich zumindest abschnittsweise um eine Längsachse der Kolbenstange (15', 15", 15'", 15"") erstrecken, zur Führung der Kolbenstange (15', 15", 15'", 15"") in dem Spritzenkörper (8) so an der Kolbenstange (15', 15", 15'", 15"") angeordnet sind, dass sie sich, wenn die Kolbenstange (15', 15", 15'", 15"") vollständig mit dem Kolben (12) verbunden ist, in dem Spritzenkörper befinden, wobei
ein Außendurchmesser (D) der drei Ringelemente (2a, 2b, 2c) gleich einem Innendurchmesser des Spritzenkörpers (8) ist oder etwas kleiner als ein Innendurchmesser des Spritzenkörpers (8) ist,
wobei zwischen den Ringelementen (2a, 2b, 2c) über den Umfang der Kolbenstange (15', 15", 15'", 15"") verteilte Flügelelemente (3a, 3b) angeordnet sind, welche sich radial nach außen erstrecken, die Ringelemente (2a, 2b, 2c) miteinander verbinden und einen Außendurchmesser (D_{F}) aufweisen, der kleiner als ein Außendurchmesser (D) der Ringelemente (2a, 2b, 2c) ist.

2. Vorgefüllte Spritze (20) nach vorstehendem Anspruch, **dadurch gekennzeichnet, dass** eine Position des Kolbens (12) in dem vorgefüllten Spritzenkörper (8) und die Position der drei Ringelemente (2a, 2b, 2c) an der Kolbenstange (15', 15", 15'", 15"") so aufeinander abgestimmt sind, dass in einem ersten Schritt, wenn die Kolbenstange (15', 15", 15'", 15"") mit ihrem Verbindungsabschnitt (1) an dem Kolben (12) angesetzt wird, bereits die beiden vorderen Ringe (2a, 2b) im Inneren des Spritzenkörpers (8) angeordnet sind.

3. Vorgefüllte Spritze (20) nach einem der vorstehenden Ansprüche, **dadurch gekennzeichnet, dass** der Außendurchmesser (D) der drei Ringelemente (2a, 2b, 2c) gleich ist.

4. Vorgefüllte Spritze (20) nach einem der vorstehenden Ansprüche, **dadurch gekennzeichnet, dass**, insbesondere jeweils, zwischen den Ringelementen (2a, 2b, 2c) mindestens vier Flügelelemente (3a, 3b), vorzugsweise unter einem Winkel von 90° zueinander, angeordnet sind.

5. Vorgefüllte Spritze (20) nach einem der vorstehenden Ansprüche, **dadurch gekennzeichnet, dass** der Außendurchmesser (D_{F}) der Flügelelemente (3a, 3b) gegenüber dem Außendurchmesser (D) der Ringelemente (2a, 2b, 2c) um 1 mm bis 10 mm, vorzugsweise um 4 mm bis 8 mm reduziert ist.

6. Vorgefüllte Spritze (20) nach einem der vorstehenden Ansprüche, **dadurch gekennzeichnet, dass** die Kolbenstange (15', 15", 15"', 15"") an dem Kolben (12) vormontiert ist.

7. Vorgefüllte Spritze (20) nach einem der vorstehenden Ansprüche, **dadurch gekennzeichnet, dass** die Kolbenstange (15', 15", 15'", 15"") neben dem Spritzenkörper (8) in einer Überverpackung liegt.

8. Vorgefüllte Spritze (20) nach vorstehendem Anspruch, **dadurch gekennzeichnet, dass** die Überverpackung eine sauerstoffimpermeable Überverpackung ist.

9. Kolbenstange (15', 15", 15'", 15"") für eine vorgefüllte Spritze (20) nach einem der vorstehenden Ansprüche, umfassend einen vorderseitigen Verbindungsabschnitt (1), über den die Kolbenstange (15', 15", 15'", 15"") mit einem Kolben (12) verbindbar ist, wobei
an einer Rückseite des Verbindungsabschnitts (1) wenigstens drei Ringelemente (2a, 2b, 2c) zur Führung der Kolbenstange (15', 15", 15'", 15"") in dem Spritzenkörper (8) angeordnet sind, welche sich zumindest abschnittsweise um eine Längsachse der Kolbenstange (15', 15", 15'", 15"") erstrecken und welche in einem Abstand R angeordnet sind mit 0,5 mm ≤ R ≤ 20mm, vorzugsweise 1 mm ≤ R ≤ 10mm.

10. Spritze (20) umfassend einen Spritzenkörper (8), einen Kolben (12) und eine Kolbenstange (15', 15", 15'", 15"") nach vorstehendem Anspruch 14, wobei der Kolben (12) so in dem Spritzenkörper (8) positionierbar ist und die drei Ringelemente (2a, 2b, 2c) so an der Kolbenstange (15', 15", 15"', 15"") angeordnet sind, dass sie sich, wenn die Kolbenstange (15', 15", 15'", 15"") vollständig mit dem Kolben (12) verbunden ist, in dem Spritzenkörper (8) befinden.

## Claims

1. A prefilled syringe (20), comprising
- a syringe body (8) which is filled with a medical fluid (10), which, on a front end, has a nozzle (9), which is closed by way of a cap (11) and is closed on a rear end by way of a displaceable plunger (12) and
- a plunger rod (15', 15", 15"', 15"") which is insertable into the syringe body (8) via the rear end, which, on a front end, has a connecting portion (1), by means of which the plunger rod (15', 15", 15'", 15"") is connectable to the plunger (12), wherein
on a rear end of the connecting portion (1) at least three ring elements (2a, 2b, 2c), which extend at least in portions about a longitudinal axis of the plunger rod (15', 15", 15'", 15""), in order to guide the plunger rod (15', 15", 15'", 15"") in the syringe body (8) are arranged on the plunger rod (15', 15", 15'", 15"") in such manner that when the plunger rod (15', 15", 15'", 15"") is fully connected to the plunger (12), it is located in the syringe body, wherein
an outside diameter (D) of the three ring elements (2a, 2b, 2c) is identical to an inside diameter of the syringe body (8) or is somewhat smaller than an inside diameter of the syringe body (8),
wherein between the ring elements (2a, 2b, 2c) wing elements (3a, 3b) distributed over the circumference of the plunger rod (15', 15", 15'", 15"") are arranged, which extend radially outwardly, connect the ring elements (2a, 2b, 2c) to one another and have an outside diameter (D_{F}), which is smaller than an outside diameter (D) of the ring elements (2a, 2b, 2c).

2. The prefilled syringe (20) according to the preceding claim, **characterized in that** a position of the plunger (12) in the prefilled syringe body (8) and the position of the three ring elements (2a, 2b, 2c) on the plunger rod (15', 15", 15'", 15"") are matched to one another such that in a first step, when the plunger rod (15', 15", 15", 15"") is placed by way of its connecting portion (1) on the plunger (12), the two front rings (2a, 2b) are arranged in the interior of the syringe body (8).

3. The prefilled syringe (20) according to one of the preceding claims, **characterized in that** the outside diameter (D) of the three ring elements (2a, 2b, 2c) is identical.

4. The prefilled syringe (20) according to one of the preceding claims, **characterized in that** in particular in each case, between the ring elements (2a, 2b, 2c) at least four wing elements (3a, 3b), are arranged preferably at an angle of 90° to one another.

5. The prefilled syringe (20) according to one of the preceding claims, **characterized in that** the outside diameter (D_{F}) of the wing elements (3a, 3b) is reduced in relation to the outside diameter (D) of the ring elements (2a, 2b, 2c) by 1 mm to 10 mm, preferably by 4 mm to 8 mm.

6. The prefilled syringe (20) according to one of the preceding claims, **characterized in that** the plunger rod (15', 15", 15"', 15"") is premounted on the plunger (12).

7. The prefilled syringe (20) according to one of the preceding claims, **characterized in that** the plunger rod (15', 15", 15'", 15"") is located next to the syringe body (8) in an external pack.

8. The prefilled syringe (20) according to the preceding claim, **characterized in that** the external pack is an oxygen-impermeable external pack.

9. A plunger rod (15', 15", 15'", 15"") for a prefilled syringe (20) according to one of the preceding claims, comprising a front-end connecting portion (1), by means of which the plunger rod (15', 15", 15'", 15"") is connectable to a plunger (12), wherein
on a rear end of the connecting portion (1) at least three ring elements (2a, 2b, 2c) are arranged to guide the plunger rod (15', 15", 15'", 15"") in the syringe body (8), which extend at least in portions about a longitudinal axis of the plunger rod (15', 15", 15'", 15"") and which are arranged at a distance R where 0.5 mm ≤ R ≤ 20 mm, preferably 1 mm ≤ R ≤ 10 mm.

10. A syringe (20), comprising a syringe body (8), a plunger (12) and a plunger rod (15', 15", 15'", 15"") according to claim 14, wherein the plunger (12) is positionable in the syringe body (8) and the three ring elements (2a, 2b, 2c) are arranged on the plunger rod (15', 15", 15"', 15"") such that when the plunger rod (15', 15", 15'", 15"") is fully connected to the plunger (12), it is located in the syringe body (8).

## Revendications

1. Seringue préremplie (20), comprenant
- un corps de seringue (8) qui est rempli d'un fluide médical (10), qui, sur une extrémité avant, a une buse (9), qui est fermée au moyen d'un capuchon (11) et est fermé sur une extrémité arrière au moyen d'un poussoir déplaçable (12) et
- une tige de piston (15', 15", 15'", 15"") qui peut être insérée dans le corps de seringue (8) par l'extrémité arrière, qui, sur une extrémité avant, a une partie de raccordement (1), au moyen de laquelle la tige de piston (15', 15", 15'", 15"") peut être raccordée au poussoir (12), dans laquelle
sur une extrémité arrière de la partie de raccordement (1) au moins trois éléments annulaires (2a, 2b, 2c), qui s'étendent au moins par parties autour d'un axe longitudinal de la tige de piston (15', 15", 15'", 15""), afin de guider la tige de piston (15', 15", 15'", 15"") dans le corps de seringue (8) sont disposés sur la tige de piston (15', 15", 15'", 15"") de telle sorte que lorsque la tige de piston (15', 15", 15'", 15"") est entièrement raccordée au poussoir (12), elle est située dans le corps de seringue, dans laquelle
un diamètre externe (D) des trois éléments annulaires (2a, 2b, 2c) est identique à un diamètre interne du corps de seringue (8) ou est légèrement plus petit qu'un diamètre interne du corps de seringue (8),
dans laquelle entre les éléments annulaires (2a, 2b, 2c) des éléments d'aile (3a, 3b) répartis sur la circonférence de la tige de piston (15', 15", 15'", 15"") sont disposés, qui s'étendent radialement vers l'extérieur, raccordent les éléments annulaires (2a, 2b, 2c) entre eux et ont un diamètre externe (D_{F}), qui est plus petit qu'un diamètre externe (D) des éléments annulaires (2a, 2b, 2c).

2. Seringue préremplie (20) selon la revendication précédente, **caractérisée en ce qu'**une position du piston (12) dans le corps de seringue prérempli (8) et la position des trois éléments annulaires (2a, 2b, 2c) sur la tige de piston (15', 15", 15'", 15"") sont appariées les unes aux autres de telle sorte que dans un premier temps, lorsque la tige de piston (15', 15", 15"', 15"") est placée au moyen de sa partie de raccordement (1) sur le poussoir (12), les deux anneaux avant (2a, 2b) sont disposés à l'intérieur du corps de seringue (8).

3. Seringue préremplie (20) selon l'une des revendications précédentes, **caractérisée en ce que** le diamètre externe (D) des trois éléments annulaires (2a, 2b, 2c) est identique.

4. Seringue préremplie (20) selon l'une des revendications précédentes, **caractérisée en ce que** notamment dans chaque cas, entre les éléments annulaires (2a, 2b, 2c) au moins quatre éléments d'aile (3a, 3b), sont disposés de préférence à un angle de 90° les uns par rapport aux autres.

5. Seringue préremplie (20) selon l'une des revendications précédentes, **caractérisée en ce que** le diamètre externe (D_{F}) des éléments d'aile (3a, 3b) est réduit par rapport au diamètre externe (D) des éléments annulaires (2a, 2b, 2c) de 1 mm à 10 mm, de préférence de 4 mm à 8 mm.

6. Seringue préremplie (20) selon l'une des revendications précédentes, **caractérisée en ce que** la tige de piston (15', 15", 15'", 15"") est prémontée sur le piston (12).

7. Seringue préremplie (20) selon l'une des revendications précédentes, **caractérisée en ce que** la tige de piston (15', 15", 15'", 15"") est située à côté du corps de seringue (8) dans un emballage externe.

8. Seringue préremplie (20) selon la revendication précédente, **caractérisée en ce que** le pack externe est un pack externe imperméable à l'oxygène.

9. Tige de piston (15', 15", 15'", 15"") pour une seringue préremplie (20) selon l'une des revendications précédentes, comprenant une partie de raccordement d'extrémité frontale (1), au moyen de laquelle la tige de piston (15', 15", 15'", 15"") peut être raccordée à un piston (12), dans laquelle sur une extrémité arrière de la partie de raccordement (1) au moins trois éléments annulaires (2a, 2b, 2c) sont agencés pour guider la tige de piston (15', 15", 15'", 15"") dans le corps de seringue (8), qui s'étendent au moins par parties autour d'un axe longitudinal de la tige de piston (15', 15", 15"', 15"") et qui sont disposés à une distance R où 0,5 mm ≤ R ≤ 20 mm, de préférence 1 mm ≤ R ≤ 10 mm.

10. Seringue (20), comprenant un corps de seringue (8), un piston (12) et une tige de piston (15', 15", 15"', 15"") selon la revendication 14, dans laquelle le piston (12) peut être positionné dans le corps de seringue (8) et les trois éléments annulaires (2a, 2b, 2c) sont disposés sur la tige de piston (15', 15", 15'", 15"") de telle sorte que lorsque la tige de piston (15', 15", 15'", 15"") est entièrement raccordée au poussoir (12), elle est située dans le corps de seringue (8).
